# EUROPEAN PATENT SPECIFICATION

(11) **EP 2 734 118 B1**
(45) Date of publication and mention of the grant of the patent: **03.05.2017**
(21) Application number: 12756029.0
(22) Date of filing: 19.07.2012
(51) Int. Cl.: A61B 8/12, A61B 18/14

(54) **ABLATION APPARATUS**
ABLATIONSVORRICHTUNG
APPAREIL D'ABLATION

(30) Priority: 22.07.2011 US 201161510599 P
(43) Date of publication of application: 28.05.2014
(73) Proprietor: Koninklijke Philips N.V., 5656 AE Eindhoven (NL)
(72) Inventor: DELADI, Szabolcs, 5656 AE Eindhoven (NL); HARKS, Godefridus Antonius, 5656 AE Eindhoven (NL)
(74) Representative: Steffen, Thomas
(86) International application number: PCT/IB2012/053692
(87) International publication number: WO 2013/014583

(56) References cited:
- EP-A2- 0 329 492
- WO-A1-2010/082146
- WO-A1-2010/131178
- WO-A1-2011/024133
- WO-A2-01/68173
- WO-A2-2010/141417
- US-A- 5 788 636
- US-A- 5 904 651
- US-A1- 2009 030 312

## Description

### FIELD OF THE INVENTION

The invention relates to an ablation apparatus, an ablation method and an ablation computer program for ablating a structure within tissue of a living being.

### BACKGROUND OF THE INVENTION

WO 2010/082146 A1 discloses a monitoring apparatus for monitoring an ablation procedure applied to an object, wherein the monitoring apparatus comprises an ultrasound signal providing unit for providing an ultrasound signal produced by sending ultrasound pulses out to the object, receiving dynamic echoes series after the ultrasound pulses have been reflected by the object, and generating the ultrasound signal depending on the received dynamic echoes series. The monitoring apparatus further comprises an ablation depth determination unit for determining an ablation depth from the provided ultrasound signal.

WO 2010/131178 A1 discloses an apparatus for determining a property of a heart, wherein the apparatus comprises a catheter including a first property sensing unit for sensing a contraction signal indicative of a reoccurring local contraction of the heart at a sensing site of the heart. The apparatus further comprises a first property determining unit for determining the reoccurring local contraction of the heart at the sensing site from the sensed contraction signal as a first property of the heart.

The adventitial tissue surrounding arteries and veins in the body contains sympathetic nerves that provide signal pathways for the regulation of hormones and proteins secreted by the cells and organs of the body. The efferent and afferent sympathetic nerves that line the renal artery are held within this adventitial connective tissue. The sympathetic nervous system is responsible for up- and down-regulation of chemicals in the body that lead to homeostasis. In the case of hypertension, the sympathetic nerves that run from the spinal cord to the kidneys signal the body to produce norepinephrine at superphysio logical levels, which leads to a cascade of signals causing a rise in blood pressure. Denervation of the renal arteries and to some extent the renal veins removes this response and allows a return to normal blood pressure.

During a renal denervation procedure by localized ablation a device is placed in the femoral artery and access to the nerves is gained through the renal artery. The nerves are embedded in the casings or layers around the renal arteries and ablated using radiofrequency energy delivery. The energy is transmitted through the vessel wall to damage the renal nerves. In this procedure, single nerves are not ablated throughout their entire length but rather at one point. The operator delivers anywhere from four to five treatments along the renal artery, distally to proximally. By placing these treatments at 12 o'clock, 3 o'clock, 6 o'clock and 9 o'clock positions, circumferentially around the renal arteries, it deactivates a significant number of the inbound and exiting renal sympathetic nerves.

The physician has no information on where the nerves are exactly located around the vessel wall during the renal denervation procedure. As a result of that, some of the nerves may not be targeted when ablating, thereby reducing the quality of the ablation procedure.

### SUMMARY OF THE INVENTION

It is an object of the present invention to provide an ablation apparatus, an ablation method and an ablation computer program for ablating a structure within tissue of a living being, wherein the quality of the ablation procedure can be improved.

In a first aspect of the present invention an ablation apparatus for ablating a nerve behind a front surface of a vessel wall within a tissue of a living being is presented, wherein the ablation apparatus comprises:
- an ultrasound visualization unit for generating a two-dimensional or three-dimensional ultrasound image of the tissue, for finding the nerve within the tissue and for generating an M-mode ultrasound image of the structure for monitoring an ablation procedure for ablating the structure within the tissue,
- an ablation unit for providing ablation energy to the tissue,
- an introduction element for introducing the ultrasound visualization unit and the ablation unit into the living being close to the tissue with the nerve to be ablated, and
- an ablation depth determination unit configured to determine an ablation depth over time from the M-mode image, wherein
- the ablation apparatus is further comprising an ultrasound control unit configured to switch from the two-dimensional or three-dimensional ultrasound imaging to M-mode ultrasound imaging when the nerve is found within the tissue, the M-mode ultrasound image comprising the nerve,
- the ablation unit is configured to apply ablation energy to the tissue after switching to M-mode imaging,
- wherein the ablation apparatus further comprises a cooling unit configured for cooling the vessel wall such that by cooling the tissue and by providing the ablation energy an intramural lesion is creatable, not extending to the front surface of the vessel wall.
The nerve is located within a wall formed by the tissue, wherein the ablation apparatus comprises a cooling unit for cooling the wall such that by cooling the tissue and by providing the ablation energy an intramural lesion is creatable, which does not extend to an outer surface of the wall, i.e. which may not extend to a front surface, through which the ablation energy is provided, and optionally also not to a back surface of the wall. Thus, a relatively small, delineated region of ablated tissue can be created within the wall, wherein the nerve to be ablated is located within this region. The tissue between this region and a front surface of the wall may not be ablated. The ablated region can therefore be more focused to the nerve, which should actually be ablated, thereby ablating less surrounding tissue. This can further improve the quality of the ablation procedure.

The cooling unit is preferentially formed by cooling fluid conduits within the introduction element for allowing cooling fluid to flow from an external cooling fluid source to the location within the living being, at which the ablation energy is provided. Since the two-dimensional or three-dimensional ultrasound image can be used for finding the nerve to be ablated within the tissue, the ablation energy can accurately be directed to the found nerve. Moreover, since during the ablation procedure the M-mode ultrasound image can be generated by sending and receiving ultrasound waves in the direction, in which the nerve is located, as found by using the two-dimensional or three-dimensional ultrasound image, the application of the ablation energy can be monitored by using the M-mode ultrasound image. This allows for an improved control of the ablation procedure and, thus, for an improved quality of this procedure.

The ultrasound image is preferentially a B-mode image. The nerve is preferentially a nerve of a renal artery, wherein the nerve is located within the vessel wall of the renal artery. The two-dimensional or three-dimensional ultrasound image is therefore preferentially used for finding the nerve of the renal artery within the vessel wall, in particular, behind a surface of the vessel wall, and the M-mode ultrasound image is preferentially used to monitor the application of the ablation energy to the nerve during the ablation procedure. This allows improving the quality of controlling the ablation of the nerve of the renal artery and, thus, the quality of the corresponding renal denervation procedure. Moreover, by combining ablation and ultrasound visualization a minimally invasive procedure for renal denervation may be performed, in particular by using the same single device, thereby allowing for a facilitated renal denervation procedure.

The ultrasound visualization unit can comprise one or several ultrasound transducers and can be adapted to directly visualize the nerve, in particular, circumferential nerves of the renal artery. It preferentially uses high frequency ultrasound for visualizing the nerve, in particular within a range of 10 to 50 MHz. The introduction element is preferentially a catheter or another hollow element being introducible into the renal artery.

The ablation apparatus further comprises an ablation depth determination unit for determining an ablation depth over time from the M-mode image and a display for displaying the determined ablation depth over time. The ablation apparatus further comprises a nerve position determination unit for determining the position of the nerve from the M-mode image and a display for displaying the determined position of the nerve. Since the absorption and scattering properties for ultrasound, in particular for high frequency ultrasound, are different for the nerve and the surrounding tissue, the position of the nerve can be determined from the M-Mode image, for instance, by thresholding. The display preferentially also shows the M-mode image, and the ablation apparatus may further comprise a wall surface determination unit for determining the position of at least one of a front surface and a back surface of a wall like a vessel wall formed by the tissue from the M-mode image, which can also be shown on the display, if the nerve is located within the wall.

The ablation depth is preferentially defined by the border between ablated tissue and non-ablated tissue in the direction of the M-mode imaging, wherein the ablation depth over time can be indicated as a line in the M-mode image. If the line indicating the ablation depth progression has completely intersected the nerve shown in the M-mode image, the nerve has been completely ablated at the respective position and the ablation can be stopped at this position.

The ultrasound visualization unit is preferentially arranged within the introduction element, which is at least partly ultrasonically transparent, for allowing the ultrasound visualization unit to perform an ultrasound visualization through the introduction element. The introduction element can comprise polyether block amide, in particular PEBAX, or polymethylpentene, in particular TPX, or another ultrasonically transparent material. In particular, a circumference of the introduction element can be ultrasonically transparent, wherein an ultrasonically transparent window can extend along the entire circumference of the introduction element. Alternatively, not the complete circumference of the introduction element may be ultrasonically transparent, but only an ultrasonically transparent window in front of the ultrasound visualization unit.

In an embodiment, the introduction element is adapted to allow a cooling fluid to flow through the introduction element and between the ultrasound visualization unit and a wall of the introduction element, which is at least partly ultrasonically transparent, for mediating the ultrasound between the ultrasound visualization unit and the wall of the introduction element. The wall of the introduction element can comprise an ablation unit like an ablation electrode, which may be attached to or incorporated in the wall of the introduction element, wherein the cooling fluid can flow between the wall and, thus, the ablation unit and the ultrasound visualization unit. In another embodiment, the ultrasound visualization unit is in direct contact with a wall of the introduction element, which is at least partly ultrasonically transparent. In this case the introduction element may not comprise a cooling fluid.

The wall of the introduction element can comprise an ablation unit like an ablation electrode, which may be attached to or incorporated in the wall of the introduction element, wherein the ultrasound visualization unit can be in direct contact with the ablation unit, which in this case is preferentially ultrasonically transparent, by gluing or depositing the ablation unit onto the ultrasound visualization unit or vice versa. The ultrasound visualization unit may also be, for instance, glued or deposited onto the ultrasonically transparent wall of the introduction element, for providing a direct contact between the wall of the introduction element and the ultrasound visualization unit, wherein the ultrasonically transparent wall may not comprise an ablation electrode, in particular if the ablation unit is adapted to optically ablate the nerve.

The ultrasound visualization unit can be rotatable within and with respect to the introduction element. In particular, the ultrasound visualization unit can be rotatable around a longitudinal axis of the introduction element. For instance, the ultrasound visualization unit can be arranged on a rotatable shaft arranged along a longitudinal axis of the introduction element. Especially in this case a circumference of the introduction element is ultrasonically transparent, in order to allow the ultrasound visualization unit to direct the ultrasound waves in a desired direction to the vessel wall.

The ablation unit and the ultrasound visualization unit are preferentially adapted such that the ablation and the ultrasound visualization are performable in the same direction. By combining ablation and ultrasound visualization such that the ablation and the visualization are performed in the same direction the respective nerve can be localized and the effect of ablation can be monitored with ultrasound, in particular in realtime, more reliably, particularly during a minimally invasive procedure for renal denervation.

It is also preferred that the ablation unit comprises an ablation electrode integrated in the introduction element and being at least partly ultrasonically transparent, wherein the ultrasound visualization unit is arranged within the introduction element such that the ultrasound visualization is performable through the ablation electrode. The ablation electrode can comprise, for instance, electrically conductive plastic or an ultrathin conductive layer like an ultrathin platinum layer for being transparent to ultrasound.

The ablation unit may comprise an ablation electrode arranged along the circumference of the introduction element. In particular, the ablation electrode maybe ultrasonically transparent along the circumference of the introduction element. Especially in this case, the ultrasound visualization unit may be rotatable within and with respect to the introduction element and the circumference of the introduction element is ultrasonically transparent, in order to allow the ultrasound visualization unit to direct the ultrasound waves in a desired direction, especially to a vessel wall containing the nerve to be ablated, and in order to allow the ablation unit to perform the ablation of the nerve in the same direction as the direction of ultrasound monitoring.

In another embodiment the ablation unit comprises at least two ablation ring electrodes attached to the introduction element, wherein the introduction element is at least partly ultrasonically transparent between the ablation ring electrodes and wherein the ultrasound visualization unit and the introduction element are arranged such that the ultrasound visualization is performable through the at least partly ultrasonically transparent introduction element between the ablation ring electrodes. The ablation ring electrodes can be bipolar radio frequency ablation rings. Also in this embodiment, the introduction element may be transparent along a circumference of the introduction element and the ultrasound visualization unit may be rotatable around a longitudinal axis of the introduction element such that ultrasound waves can be transmitted into the tissue, especially into a vessel wall containing the nerve, in a desired radial direction.

It is further preferred that the introduction element comprises a transparent region being ultrasonically and optically transparent, wherein the ablation unit comprises an optical element for providing ablation light for ablating the nerve, wherein the introduction element, the ultrasound visualization unit and the optical element are arranged such that ultrasound waves of the ultrasound visualization unit and the ablation light are transmittable through the transparent region. The transparent region can comprise, for instance, PEBAX.

It is also preferred that the ultrasound visualization unit is adapted to allow the ablation light to traverse the ultrasound visualization unit, wherein the ultrasound visualization unit and the optical element are arranged such that the ablation light is transmittable through the ultrasound visualization unit. For instance, the ultrasound visualization unit can comprise an opening through which the ablation light is transmittable. The opening can be provided by materializing a nozzle in the ultrasound visualization unit. Alternatively or in addition, the ultrasound visualization unit may comprise optically transparent material for allowing the ablation light to be transmitted through the ultrasound visualization unit.

There can be a gap between the ultrasound visualization unit and the transparent region of the introduction element, in order to allow cooling fluid to flow in between the gap. However, the ultrasound visualization unit can also be in direct contact with the transparent region, wherein cooling fluid may not flow through the introduction element.

The optical element can be, for instance, an optical fiber in combination with an outcoupling mirror for guiding the ablation light to a desired location within the living being, particularly within a renal artery, and for coupling the light out of the optical fiber.

It is further preferred that the introduction element includes a sheath to be introduced into the living being, wherein the sheath comprises the ablation unit and is at least partly ultrasonically transparent, and wherein the ultrasound visualization unit is arranged within the sheath such that ultrasound waves of the ultrasound visualization unit are transmittable through the at least partly ultrasonically transparent sheath. The ablation unit may comprise ablation ring electrodes on the outer surface of the sheath, wherein the ultrasound visualization unit can be arranged within the sheath such that ultrasound sensing can be performed through a gap between the ablation ring electrodes, at which the sheath is ultrasonically transparent. Alternatively or in addition, the ablation unit may comprise an ultrasonically transparent electrode forming a part of the sheath, wherein the ultrasound visualization unit can be arranged within the sheath such that the ultrasound sensing can be performed through the ultrasonically transparent electrode. The ultrasound visualization unit can be an ultrasound visualization catheter arrangable within the sheath, wherein the ultrasound visualization catheter can be, for instance, an intravascular ultrasound (IVUS) catheter.

In a further aspect of the present invention an ablation computer program for ablating a nerve behind a front surface of a vessel wall within a tissue of a living being is presented, the computer program comprising program code means for causing an ablation apparatus as defined in claim 1 to be operated such that the two-dimensional or three-dimensional ultrasound imaging switches to M-mode ultrasound imaging when the nerve is found within the tissue, the M-mode ultrasound image comprising the nerve, the ablation unit applies ablation energy to the tissue after switching to M-mode imaging, and the ablation apparatus is cooling the vessel wall such that by cooling the tissue and by providing the ablation energy an intramural lesion is creatable, not extending to the front surface of the vessel wall, when the computer program is run on a computer controlling the ablation apparatus.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the drawings:
Fig. 1 shows schematically and exemplarily an embodiment of an ablation apparatus for ablating a structure within tissue of a living being,
Fig. 2 shows schematically and exemplarily a progressing ablation line, front and back surfaces of a vessel wall and a nerve within the vessel wall, which may be shown on a display of the ablation apparatus,
Figs. 3 to 9 schematically and exemplarily show different embodiments of an introduction element for being introduced into the renal artery,
Fig. 10 shows schematically and exemplarily an intramural lesion within a vessel wall of a renal artery, wherein the intramural lesion covers a nerve within the vessel wall for ablating the nerve, and
Fig. 11 shows a flowchart exemplarily illustrating an embodiment of an ablation method for ablating a structure within tissue of a living being.

### DETAILED DESCRIPTION OF EMBODIMENTS

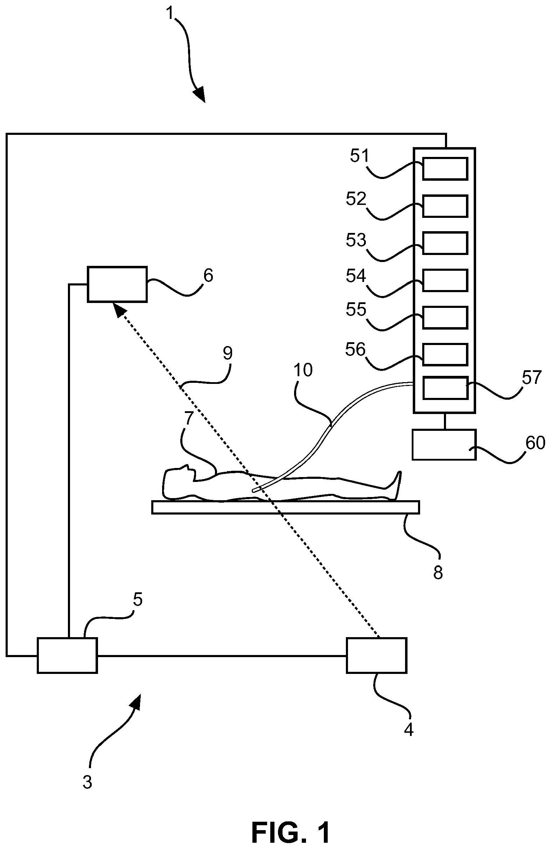

Fig. 1 shows schematically and exemplarily an ablation apparatus 1 for ablating a structure within tissue of a living being, which is, in this embodiment, an ablation apparatus for ablating a nerve of a renal artery and which comprises an introduction element 10 for introducing an ultrasound visualization unit and an ablation unit into the renal artery. The introduction element 10 is preferentially a catheter or another hollow element being introducible into the renal artery of the person 7 lying on a table 8. The ultrasound visualization unit is an ultrasound transducer for ultrasonically visualizing the nerve of the renal artery before and during an ablation procedure and the ablation unit comprises an ablation electrode for ablating the nerve of the renal artery.

The ultrasound visualization unit is adapted to use high frequency ultrasound for visualizing the nerve, wherein a frequency between 10 and 50 MHz is used. It is controlled by an ultrasound control unit 54 such that the ultrasound visualization unit generates firstly a B-mode ultrasound image for finding the nerve behind a surface of a vessel wall of the renal artery and to generate an M-mode ultrasound image of the nerve for monitoring the ablation of the nerve. Thus, a B-mode image can be used for finding the nerve behind the surface of the vessel wall of the renal artery, wherein, after the nerve has been found, the ultrasound visualization unit can be switched to M-mode ultrasound imaging by the ultrasound control unit 54, in order to perform an M-mode imaging through the position of the nerve for monitoring the effect of ablation, wherein, after the M-mode monitoring has been started, also the ablation can be started.

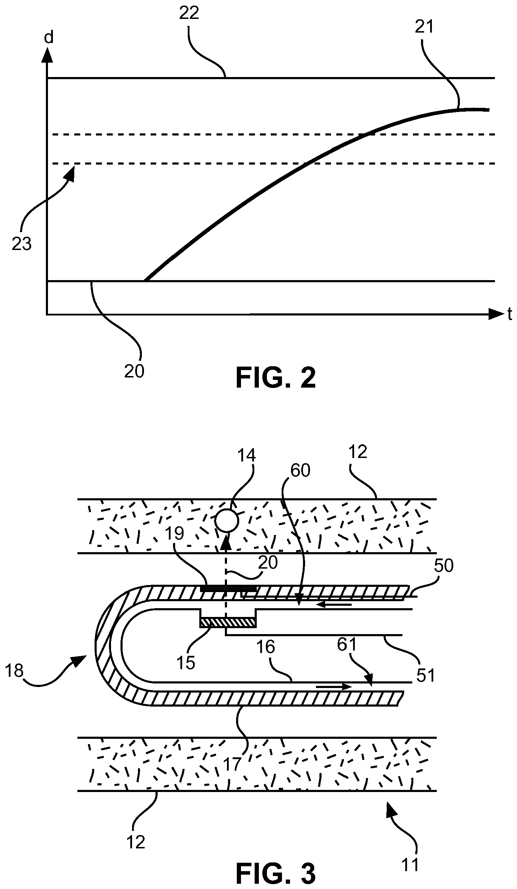

The ablation apparatus 1 further comprises an ablation depth determination unit 51 for determining an ablation depth over time from the M-mode image, a structure position determination unit being, in this embodiment, a nerve position determination unit 52 for determining the position of the nerve from the M-mode image, and a wall surface determination unit 56 for determining the position of a front surface and a back surface of the vessel wall. The ablation apparatus 1 also comprises a display 60 for displaying the determined ablation depth progressing over time, the determined position of the nerve and the determined positions of the front surface and the back surface of the vessel wall. Fig. 2 shows schematically and exemplarily the determined ablation depth and the determined positions as they may be shown on the display 60. In Fig. 2 the ablation depth 21 over time is indicated by the line 21, the position of the front surface of the vessel wall is indicated by the line 20, the position of the back surface of the vessel wall is indicated by the line 22 and the position of the nerve is indicated by broken lines 23. In Fig. 2 these characteristics are shown at the respective depth position *d* depending on the time *t.* The different lines shown in Fig. 2 can be overlayed with the M-mode image on the display 60.

The ablation depth is defined by the border between ablated tissue and non-ablated tissue in the direction of the M-mode imaging, wherein, when the line 21 indicating the ablation depth progression has completely intersected the nerve indicated by the broken lines 23, the nerve has been completely ablated at the respective M-mode monitoring position and the ablation can be stopped. This stopping can be performed manually by a user like a physician watching the display 60 or automatically.

The ablation depth determination unit 51, the nerve position determination unit 52 and the wall surface determination unit 55 can be adapted to use known detection algorithms for detecting the respective characteristics based on the generated M-mode image. For instance, the detection algorithms disclosed in US 2012/0004547 A1 can be used for detecting the respective characteristics based on the generated M-mode image.

Fig. 3 shows schematically and exemplarily an embodiment of a tip 18 of the catheter 10 after having been introduced into the renal artery 11 with the vessel wall 12 and the nerve 14. It should be noted that Fig. 3 and also Figs. 1, 2 and 4 to 10 are not to scale.

The catheter comprises an outer tube 17 being ultrasonically transparent and an inner central element 16 with an ultrasound transducer 15 forming the ultrasound visualization unit. The ultrasound transducer 15 can have a one-dimensional or a two-dimensional array of ultrasound elements. It is connected with the ultrasound control unit 54 via an electrical connection 51. An inflow channel 60 and an outflow channel 61 are provided for allowing cooling fluid to flow within the introduction element. The inner central element 16 can be glued to the outer tube 17 proximal to the tip 18 shown in Fig. 3 such that the cooling fluid can circulate around the ultrasound transducer 15. The outer tube 17 can comprise at its proximal end an inlet and an outlet for the cooling fluid.

An ultrasonically transparent ablation electrode 19 is provided on the outer tube 17 at a position, at which ultrasound waves 20 of the ultrasound transducer 15 transmit through the outer tube 17. Thus, the ultrasound visualization unit 15 is arranged within the introduction element, which is at least partly ultrasonically transparent, for allowing the ultrasound visualization unit 15 to perform ultrasound visualization through the introduction element. The ultrasonically transparent outer shell 17 can comprise PEBAX, TPX or another ultrasonically transparent material. The ablation electrode 19 can comprise, for instance, electrically conductive plastic, an ultrathin conductive layer like an ultrathin platinum layer or another electrically conducting ultrasonically transparent material.

The ablation electrode is electrically connected to an ablation control unit 56 being, in this embodiment, a radio frequency source for performing a radio frequency ablation procedure via the ablation electrode 19. The electrical connection 50 is provided by wires.

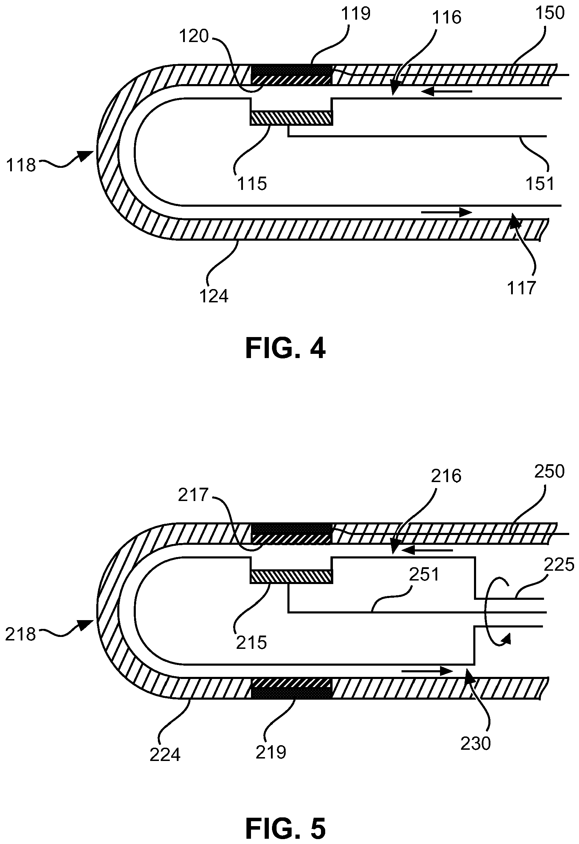

The outer tube of the introduction element can be ultrasonically transparent around the entire circumference of the introduction element. However, in an embodiment not the complete circumference of the introduction element may be ultrasonically transparent, but only a smaller ultrasonically transparent window in front of the ultrasound visualization unit, in particular, in front of the ultrasound transducer as schematically and exemplarily shown in Fig. 4.

In Fig. 4 the tip 118 of the introduction element comprises an outer tube 124, which can also be regarded as being an outer shell, which is only ultrasonically transparent in front of the ultrasound transducer 115, which is connected with the ultrasound control unit 54 via an electrical connection 151. The outer tube 124 comprises therefore an ultrasonically transparent window 120 in front of the ultrasound transducer 115. An ultrasonically transparent ablation electrode 119 is provided on the ultrasonically transparent window 120, in order to provide ultrasound sensing and ablation in the same direction. The ablation electrode 119 is connected to the ablation control unit 56 via the electrical connection 150. An inflow channel 116 and an outflow channel 117 are provided for allowing cooling fluid to flow within the introduction element. Fig. 4 shows therefore a device with internal cooling, wherein the cooling fluid mediates an ultrasound transducer contact with the ultrasonically transparent ablation electrode.

In another embodiment the introduction element may not comprise internal cooling fluid, wherein an ultrasonically transparent ablation electrode may be in direct contact with the ultrasound transducer. Or, if in an embodiment an ultrasonically transparent ablation electrode is not used, but another kind of ablation electrode or another ablation unit providing another kind of ablation energy like optical ablation energy, the ultrasound transducer may be in direct contact with a wall of the introduction element, for instance, with an outer tube of the introduction element, which is at least partly ultrasonically transparent. The ultrasound transducer may be glued to or deposited on the wall of the introduction element or it may be provided to the wall of the introduction element in another way.

Thus, the wall of the introduction element can comprise an ablation unit like an ablation electrode, which may be attached to or incorporated in the wall of the introduction element, wherein the ultrasound transducer can be in direct contact with the ablation unit, which in this case is preferentially ultrasonically transparent, by, for instance, gluing or depositing the ablation unit onto the ultrasound transducer or vice versa. The ultrasound transducer may also be, for instance, glued or deposited onto the ultrasonically transparent wall of the introduction element for providing a direct contact between the wall of the introduction element and the ultrasound transducer, wherein the ultrasonically transparent wall may not comprise an ablation electrode, in particular, if the ablation unit is adapted to optically ablate the nerve.

The ultrasound transducer maybe rotatable within and with respect to the introduction element 10 as schematically and exemplarily shown in, for instance, Fig. 5. In Fig. 5, the ultrasound transducer 215, which is electrically connected to the ultrasound control unit 54 via an electrical connection 251, is rotatable around a longitudinal axis of the introduction element. The ultrasound transducer 215 is arranged on a rotatable shaft 225 arranged along the longitudinal axis of the introduction element. The tip 218 of the introduction element comprises an outer tube 224 with an ultrasonically transparent cylindrical window 217, i.e. the circumference of the introduction element at the position of the ultrasound transducer 215 is ultrasonically transparent, in order to allow the ultrasound transducer 215 to direct the ultrasound waves in a desired direction to the vessel wall. Also in this embodiment the introduction element comprises an inflow channel 216 and an outflow channel 230 for providing cooling fluid. Fig. 5 shows therefore a device with internal cooling, wherein the fluid mediates the ultrasound transducer contact with the ultrasonically transparent ablation electrode 219, which may be cylindrically provided on the ultrasonically transparent cylindrical window 217 and which is connected to the ablation control unit 56 via an electrical connection 250. Thus, in this embodiment the outer tube 224 comprises an ultrasonically transparent cylindrical window and is provided with an ultrasonically transparent cylindrical ablation electrode, in order to allow the ultrasound transducer, which is rotatably attached to the shaft 225, to direct ultrasound waves in a desired direction to the vessel wall and in order to allow the ablation unit to perform the ablation of the nerve in the same direction.

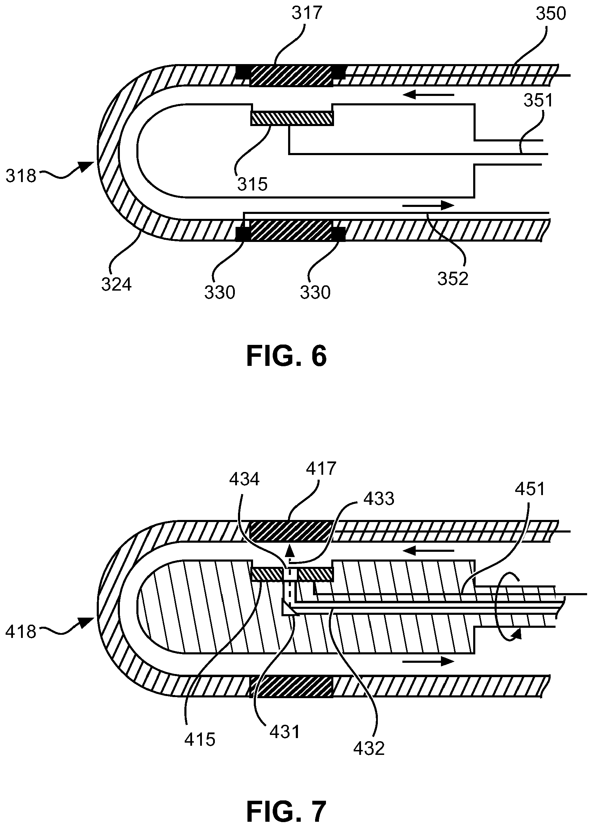

Fig. 6 shows schematically and exemplarily a further embodiment of a tip 318 of the introduction element, which, regarding the internal cooling and the rotatable ultrasound transducer, is similar to the embodiment shown in Fig. 5. However, the embodiment schematically and exemplarily shown in Fig. 6 comprises two bipolar radio frequency ablation rings 330 as the ablation unit, which are attached to the introduction element and which are electrically connected to the ablation control unit 56 via electrical connections 350, 352, wherein the outer tube 324 of the introduction element is at least partly ultrasonically transparent between the ablation ring electrodes 330. The ultrasound transducer 315, which is attached to a rotatable shaft 325 and which is electrically connected to the ultrasound control unit 54 via an electrical connection 351, and the introduction element, in particular, the outer tube 324, are arranged such that the ultrasound visualization is performable through the at least partly ultrasonically transparent introduction element between the ablation ring electrodes 330. Thus, between the ablation ring electrodes 330 an ultrasonically transparent window 317 is provided, through which the ultrasound sensing can be performed. The ablation ring electrodes 330 may be metal electrodes like platinum electrodes.

Fig. 7 shows schematically and exemplarily a further embodiment of a tip 418 of the introduction element. As can be seen in Fig. 7, the introduction element may comprise a transparent region 417, which may be cylindrically arranged around an ultrasound transducer 415 and which may be ultrasonically and optically transparent. In this embodiment the ablation unit comprises an optical element being a combination of an optical fiber 432 for providing laser ablation light 433 and an outcoupling mirror 431 for directing the ablation light 433 to the nerve 14. The introduction element, the ultrasound transducer 415, which is electrically connected to the ultrasound control unit 54 via an electrical connection 451, and the optical element 431, 432 are arranged such that ultrasound waves of the ultrasound transducer 415 and the ablation light 433 are transmittable through the transparent region 417. The transparent region 417 can comprise, for instance, PEBAX. The inner element 416 with the ultrasound transducer 415 and the optical elements 431, 432 are rotatable with respect to the introduction element.

The ultrasound transducer 415 is adapted to allow the ablation light 433 to traverse the ultrasound transducer 415 such that the ablation light 433 is transmittable through the ultrasound transducer 415. In this embodiment, the ultrasound transducer 415 comprises an opening 434 like a nozzle through which the ablation light 433 is transmittable. Alternatively or in addition, the ultrasound transducer can comprise optically transparent material for allowing the ablation light to be transmitted through the ultrasound transducer.

There is a gap between the ultrasound transducer 415 and the transparent region 417 of the introduction element, in order to allow cooling fluid to flow in between the gap. Thus, an internal cooling can be provided, wherein the fluid mediates ultrasound transducer contact with the outer wall of the introduction element.

In another embodiment, the ultrasound transducer can also be in direct contact with the transparent region. For instance, the ultrasound transducer can be glued, deposited or provided in another way on the transparent region, wherein also in this case the ablation source can be optical energy, in particular laser energy, fed in the introduction element via an optical fiber and coupled out via a mirror.

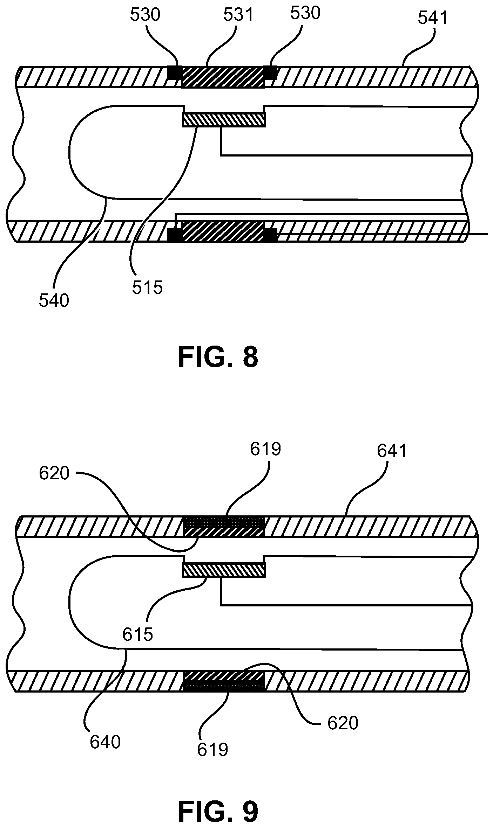

Fig. 8 shows schematically and exemplarily a part of a further embodiment of the introduction element. In this embodiment the introduction element includes a sheath 541 to be introduced into the renal artery 11, wherein the sheath 541 comprises the ablation unit 530 and is at least partly ultrasonically transparent. The ultrasound visualization unit is, in this embodiment, an ultrasound visualization catheter 540 with an ultrasound transducer 515. The ultrasound catheter 540 is, for instance, an IVUS catheter arranged within the sheath 541 such that ultrasound waves of the ultrasound visualization catheter 540 are transmittable through the at least partly ultrasonically transparent sheath 541. In this embodiment the ablation unit comprises ablation ring electrodes 530 on the outer surface of the sheath 541, which can be used for performing a bipolar radio frequency ablation. The ultrasound catheter 540 is arranged within the sheath 541 such that ultrasound sensing can be performed through a gap 531 between the ablation electrodes 530, at which the sheath 541 is ultrasonically transparent.

Fig. 9 shows schematically and exemplarily a further part of an embodiment of the introduction element. Regarding the ultrasound visualization unit arranged within a sheath comprising an ultrasonically transparent window such that the ultrasound sensing can be provided through the sheath, the embodiment shown in Fig. 9 is similar to the embodiment shown in Fig. 8. In particular, also in this embodiment an ultrasound visualization catheter 640 with an ultrasound transducer 615 is arranged within a sheath 641 being at least partly ultrasonically transparent, in order to allow ultrasound sensing through the sheath 641. However, in this embodiment the sheath 641 does not comprise ablation ring electrodes, but the ablation unit comprises an ultrasonically transparent electrode 619 forming a part of the sheath 641, wherein the ultrasound transducer 615 can be arranged within the sheath 641 such that the ultrasound sensing can be performed through the ultrasonically transparent electrode 619. In this embodiment the ultrasonically transparent ablation electrode 619 and a corresponding ultrasonically transparent window 620 of the sheath 641 are arranged on the whole circumference of the sheath 641. In another embodiment, they can also be arranged on a part of the circumference of the sheath 641.

The ablation apparatus 1 further comprises a localization unit 3 for localizing the introduction element 10 within the person 7. The localization unit 3 comprises is, in this embodiment, an x-ray fluoroscopy system with an x-ray source 4 and an x-ray detector 6. The x-ray source 4 emits an x-ray beam 9 which traverses the person 7 including the tip of the introduction element 10. The x-ray beam, which has traversed the person 7, is detected by the x-ray detector 6. The x-ray detector 6 generates electrical signals depending on the detected x-ray beam and the electrical signals are used by a fluoroscopy control unit 5 for generating an x-ray projection image. The fluoroscopy control unit 28 is also adapted to control the x-ray source 4 and the x-ray detector 6. The x-ray source 4 and the x-ray detector 6 can be adapted to be rotatable around the person 7 for allowing the x-ray fluoroscopy system to generate x-ray projection images in different directions. The x-ray fluoroscopy system is, for example, a computed tomography fluoroscopy system or a C-arm fluoroscopy system. The x-ray projection images can be shown on the display 60 for allowing a user like a physician to navigate the introduction element within the person 7 depending on the x-ray projection images shown on the display 60.

In other embodiments, the localization unit can comprise other means like a magnetic resonance imaging system or location sensors, for example, for magnetic and/or impedance based tracking at the distal end of the introduction element for determining the position and optionally also the orientation of the introduction element. In further embodiments, the localization unit may comprise sensors for optical shape sensing, for example, based on fiber Bragg gratings or Rayleigh scattering.

The ablation apparatus 1 further comprises a navigation unit 53 for allowing the introduction element 10 to be navigated to a desired location within the person 7. The navigation unit 53 can be adapted to allow a user to navigate the introduction element 10 completely by hand or semi-automatically. The introduction element 10 comprises built-in guiding means (not shown in Fig. 1), which can be controlled by the navigation unit 53. The introduction element 10 can, for example, be steered and navigated by the use of steering wires in order to guide the tip of the introduction element 10 to a desired location within the person 7. The ablation apparatus 1 further comprises a cooling fluid source 57 for providing the cooling fluid for cooling the tissue and the structure to be ablated. The cooling fluid source 57 together with cooling fluid conduits within the introduction element can be regarded as being a cooling unit for cooling the tissue and the structure to be ablated.

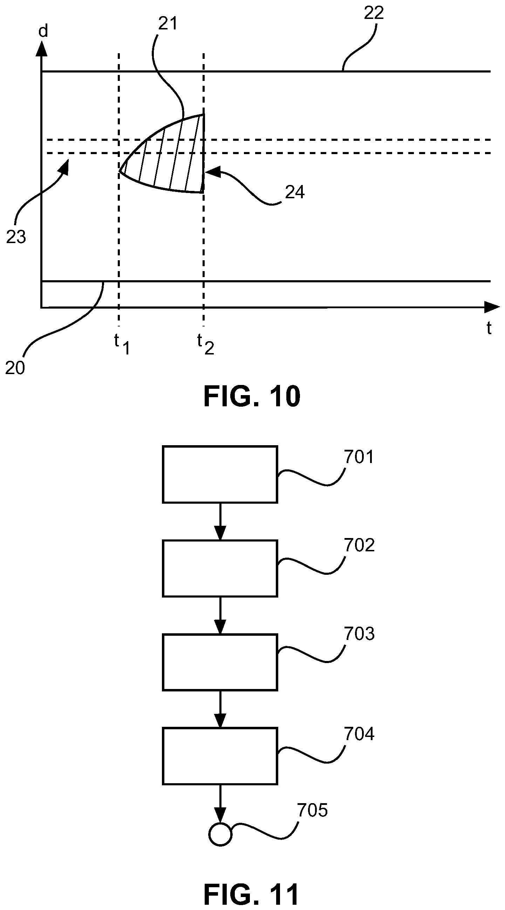

The ablation unit and cooling unit can be adapted such that the combination of the provisions of the ablation energy and the cooling leads to an intramural lesion. Thus, through the circulation of the cooling fluid an intramural lesion can be formed, which spares a front part of the vessel wall, in particular, the front surface of the vessel wall, as schematically and exemplarily shown in Fig. 10.

In Fig. 10, the line 21 indicates the border between the ablated region of the vessel wall and the remaining, non-ablated parts of the vessel wall, i.e. it defines the border between ablated tissue and non-ablated tissue and can be regarded as being a lesion delimiting line. Moreover, similar to Fig. 2, reference number 22 indicates the position of the back surface of the vessel wall and reference number 20 indicates the position of the front surface of the vessel wall. The horizontal broken lines 23 indicate the position of the nerve. In this example, the ablation procedure started at *t*₁ and the ablation procedure stopped at *t*₂ such that an intramural lesion indicated by the solid line 24 is created.

The different lines 20...24 can be determined by the ablation depth determination unit 51, the structure position determination unit 52 and the wall surface determination unit 56 described above, in particular, with reference to Fig. 2. In this embodiment, the ablation depth determination unit 51 determines for each point in time between *t*₁ and *t*₂ two ablation depths, i.e. the respective borders non-ablated/ablated tissue and ablated/non-ablated tissue.

In the following an ablation method for ablating a structure within tissue of a living being, which is, in this embodiment, an ablation method for ablating a nerve of a renal artery, will exemplarily be described with reference to a flowchart shown in Fig. 10.

In step 701 an ultrasound visualization unit and an ablation unit are introduced into a renal artery by using an introduction element. In step 702 the ultrasound visualization unit is controlled to perform a B-mode imaging for finding the nerve to be ablated in the vessel wall of the renal artery. In particular, the ultrasound visualization unit can be moved within the renal artery, until the nerve to be ablated is visible in the B-mode image. Then, in step 703 the nerve of the renal artery can be ultrasonically visualized by generating an M-mode image. Thus, the ultrasound visualization unit can be switched to an M-mode imaging mode for monitoring the ablation procedure. While the M-mode image is continuously generated, the ablation of the nerve of the renal artery is started and performed by using an ablation unit in step 704. During the ablation procedure the ablation depth can be determined and shown on a display together with the M-mode image. Moreover, the position of the nerve within the vessel wall can be determined and shown on the display. After the progressing ablation depth shown on the display has completely intersected the nerve to be ablated, the ablation method can be manually or automatically stopped in step 705.

During a renal denervation procedure by localized ablation a device is placed in the femoral artery and access to the nerves is gained through the renal artery. The nerves are embedded in the casings or layers around the renal arteries and ablated using radiofrequency energy delivery. The energy is transmitted through the vessel wall to damage the renal nerves. In this procedure, single nerves are not ablated throughout their entire length but rather at one point. The operator delivers anywhere from four to five treatments along the renal artery, distally to proximally. By placing these treatments at the 12 o'clock, 3 o'clock, 6 o'clock and 9 o'clock positions, circumferentially around the renal arteries, it deactivates a significant number of the inbound and exiting renal sympathetic nerves.

In known renal denervation procedures the physician has no information on where the nerves are exactly located around the vessel wall. As a result of that, some of the nerves may not be targeted when ablating according to the clockwise approach. Also the effect of renal denervation on the systemic blood pressure is not instantaneous (but takes minutes to days) and it would therefore be very helpful if changes in nerve functionality and/or integrity could be assessed during or immediately after radio frequency energy delivery. The ablation apparatus and ablation method described above provide therefore an ultrasound imaging technique, which allows monitoring the ablation of the nerve very accurately, thereby improving the quality of the renal denervation procedure.

Although in Figs. 3 to 9 certain arrangements of different elements have been shown, also other arrangements of the ultrasound visualization unit, the ablation unit and the introduction element are possible. Also the wiring can be different. For instance, the wiring for the ablation unit may not be located in a wall of the introduction element, but, for instance, in the space within the introduction element. Moreover, also the wiring may be at least partly ultrasonically transparent, in particular in the embodiments shown in Figs. 6 and 8.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Determinations like the determination of the ablation depth, the determination of the position of the structure, the determination of the position of at least one of the front surface and the back surface of the wall, et cetera performed by one or several units or devices can also be performed by any other number of units or devices. The determinations and/or the control of the ablation apparatus in accordance with the ablation method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an an ablation apparatus for ablating a structure within tissue of a living being. An ultrasound visualization unit generates a two-dimensional or a three-dimensional ultrasound image for finding the structure to be ablated within the tissue. Ablation energy provided by an ablation unit can then accurately be directed to the found structure. Moreover, the ultrasound visualization unit is adapted to generate during the provision of the ablation energy an M-mode ultrasound image by sending and receiving ultrasound waves in the direction, in which the structure is located, as found by using the two-dimensional or three-dimensional ultrasound image, thereby monitoring the ablation procedure. This allows for an improved control of the ablation procedure and, thus, for an improved quality of this procedure.

## Claims

1. An ablation apparatus for ablating a nerve behind a front surface of a vessel wall within a tissue of a living being, the ablation apparatus comprising:
- an ultrasound visualization unit (15; 115; 215; 315; 415; 515; 615) for generating a two-dimensional or three-dimensional ultrasound image of the tissue, for finding the nerve (14) within the tissue and for generating an M-mode ultrasound image of the structure (14) for monitoring an ablation procedure for ablating the structure within the tissue,
- an ablation unit (19; 119; 219; 330; 431, 432; 530; 619) for providing ablation energy to the tissue,
- an introduction element (10) for introducing the ultrasound visualization unit (15; 115; 215; 315; 415; 515; 615) and the ablation unit (19; 119; 219; 330; 431, 432; 530; 619) into the living being (7) close to the tissue with the nerve (14) to be ablated, and
- an ablation depth determination unit (51) configured to determine an ablation depth over time from the M-mode image,
**characterized in that**
- the ablation apparatus is further comprising an ultrasound control unit (54) configured to switch from the two-dimensional or three-dimensional ultrasound imaging to M-mode ultrasound imaging when the nerve is found within the tissue, the M-mode ultrasound image comprising the nerve,
- the ablation unit is configured to apply ablation energy to the tissue after switching to M-mode imaging,
- wherein the ablation apparatus further comprises a cooling unit configured for cooling the vessel wall such that by cooling the tissue and by providing the ablation energy an intramural lesion is creatable, not extending to the front surface of the vessel wall.

2. The ablation apparatus as defined in claim 1, wherein the ultrasound visualization unit (215; 315) is rotatable within and with respect to the introduction element (10).

3. The ablation apparatus as defined in claim 1, wherein the ablation unit and the ultrasound visualization unit are adapted such that the ablation and the ultrasound visualization are performable in the same direction.

4. The ablation apparatus as defined in claim 3, wherein the ablation unit comprises an ablation electrode (19; 119; 219; 619) integrated in the introduction element and being at least partly ultrasonically transparent and wherein the ultrasound visualization unit (15; 115; 215; 615) is arranged within the introduction element such that the ultrasound visualization is performable through the ablation electrode (19; 119; 219; 619).

5. The ablation apparatus as defined in claim 1, wherein the introduction element comprises a transparent region (417) being ultrasonically and optically transparent, wherein the ablation unit comprises an optical element (431, 432) for providing ablation light (433) for ablating the nerve (14), wherein the introduction element, the ultrasound visualization unit (415) and the optical element (431, 432) are arranged such that ultrasound waves of the ultrasound visualization unit (415) and the ablation light (433) are transmittable through the transparent region (417).

6. The ablation apparatus as defined in claim 5, wherein the ultrasound visualization unit (415) is adapted to allow the ablation light (433) to traverse the ultrasound visualization unit (415), wherein the ultrasound visualization unit (415) and the optical element (431, 432) are arranged such that the ablation light (433) is transmittable through the ultrasound visualization unit (415).

7. The ablation apparatus as defined in claim 6, wherein the ultrasound visualization unit (415) comprises an opening (434) through which the ablation light (433) is transmittable.

8. The ablation apparatus as defined in claim 1, wherein
- the ultrasound visualization unit (15; 115; 215; 315; 415; 515; 615) is adapted to generate a two-dimensional or three-dimensional ultrasound image for finding a nerve (14) of a renal artery (11) behind a surface of a vessel wall of the renal artery (11) and to generate an M-mode ultrasound image of the nerve (14) for monitoring the ablation procedure,
- the ablation unit (19; 119; 219; 330; 431, 432; 530; 619) is adapted to ablate the nerve (14) of the renal artery (11),
- the introduction element (10) is adapted to introduce the ultrasound visualization unit (15; 115; 215; 315; 415; 515; 615) and the ablation unit (19; 119; 219; 330; 431, 432; 530; 619) into the renal artery (11).

9. The ablation apparatus as defined in claim 1, wherein the introduction element includes a sheath (541; 641) to be introduced into the living being (7), wherein the sheath (541; 641) comprises the ablation unit (530; 619) and is at least partly ultrasonically transparent, and wherein the ultrasound visualization unit (515; 615) is arranged within the sheath (541; 641) such that ultrasound waves of the ultrasound visualization unit (515; 615) are transmittable through the at least partly ultrasonically transparent sheath (541; 641).

10. The ablation apparatus as defined in claim 10, wherein the ultrasound visualization unit is an ultrasound visualization catheter arrangable within the sheath.

11. An ablation computer program for ablating a nerve behind a front surface of a vessel wall within a tissue of a living being, **characterized in that** the computer program comprises program code means for causing an ablation apparatus as defined in claim 1 to be operated such that the two-dimensional or three-dimensional ultrasound imaging switches to M-mode ultrasound imaging when the nerve is found within the tissue, the M-mode ultrasound image comprising the nerve, the ablation unit applies ablation energy to the tissue after switching to M-mode imaging, and the ablation apparatus is cooling the vessel wall such that by cooling the tissue and by providing the ablation energy an intramural lesion is creatable, not extending to the front surface of the vessel wall, when the computer program is run on a computer controlling the ablation apparatus.

## Patentansprüche

1. Ablationsvorrichtung zum Abladieren eines Nervs hinter einer Vorderfläche einer Gefäßwand innerhalb eines Gewebes eines Lebewesens, wobei die Ablationsvorrichtung Folgendes umfasst:
- eine Ultraschallvisualisierungseinheit (15; 115; 215; 315; 415; 515; 615) zum Erzeugen eines zweidimensionalen oder dreidimensionalen Ultraschallbilds des Gewebes, um den Nerv (14) innerhalb des Gewebes zu finden, und zum Erzeugen eines M-Mode-Ultraschallbilds der Struktur (14), um eine Ablationsprozedur zum Abladieren der Struktur innerhalb des Gewebes zu überwachen,
- eine Ablationseinheit (19; 119; 219; 330; 431, 432; 530; 619) zum Bereitstellen von Ablationsenergie für das Gewebe;
- ein Einführungselement (10) zum Einführen der Ultraschallvisualisierungseinheit (15; 115; 215; 315; 415; 515; 615) und der Ablationseinheit (19; 119; 219; 330; 431, 432; 530; 619) in das Lebewesen (7) nahe dem Gewebe mit dem zu abladierenden Nerv (14), und
- eine Ablationstiefe-Ermittlungseinheit (51), die konfiguriert ist, um eine Ablationstiefe über die Zeit anhand des M-Mode-Bilds zu ermitteln,
**dadurch gekennzeichnet, dass**
- die Ablationsvorrichtung weiterhin eine Ultraschallsteuereinheit (54) umfasst, die konfiguriert ist, um von der zweidimensionalen oder dreidimensionalen Ultraschallbildgebung auf M-Mode-Ultraschallbildgebung umzuschalten, wenn der Nerv in dem Gewebe gefunden wurde, wobei das M-Mode-Ultraschallbild den Nerv umfasst,
- die Ablationseinheit konfiguriert ist, um Ablationsenergie an das Gewebe abzugeben, nachdem auf die M-Mode-Bildgebung umgeschaltet wurde,
- wobei die Ablationsvorrichtung weiterhin eine Kühleinheit umfasst, die konfiguriert ist, um die Gefäßwand zu kühlen, so dass durch Kühlen des Gewebes und durch Bereitstellen der Ablationsenergie eine intramurale Läsion erzeugbar ist, die sich nicht zu der Vorderfläche der Gefäßwand erstreckt.

2. Ablationsvorrichtung nach Anspruch 1, wobei die Ultraschallvisualisierungseinheit (215; 315) innerhalb des Einführungselements (10) und in Bezug darauf drehbar ist.

3. Ablationsvorrichtung nach Anspruch 1, wobei die Ablationseinheit und die Ultraschallvisualisierungseinheit derartig ausgelegt sind, dass die Ablation und die Ultraschallvisualisierung in der gleichen Richtung durchführbar sind.

4. Ablationsvorrichtung nach Anspruch 3, wobei die Ablationseinheit eine Ablationselektrode (19; 119; 219; 619) umfasst, die in das Einführungselement integriert ist und zumindest teilweise für Ultraschall transparent ist, und wobei die Ultraschallvisualisierungseinheit (15; 115; 215; 615) derartig in dem Einführungselement angeordnet ist, dass die Ultraschallvisualisierung durch die Ablationselektrode (19; 119; 219; 619) durchführbar ist.

5. Ablationsvorrichtung nach Anspruch 1, wobei das Einführungselement eine transparente Region (417) umfasst, die für Ultraschall und optisch transparent ist, wobei die Ablationseinheit ein optisches Element (431, 432) zum Bereitstellen von Ablationslicht (433) zum Abladieren des Nervs (14) umfasst, wobei das Einführungselement, die Ultraschallvisualisierungseinheit (415) und das optische Element (431, 432) derartig angeordnet sind, dass Ultraschallwellen der Ultraschallvisualisierungseinheit (415) und das Ablationslicht (433) durch die transparente Region (417) übertragbar sind.

6. Ablationsvorrichtung nach Anspruch 5, wobei die Ultraschallvisualisierungseinheit (415) dafür ausgelegt ist, dem Ablationslicht (433) zu erlauben, die Ultraschallvisualisierungseinheit (415) zu durchqueren, wobei die Ultraschallvisualisierungseinheit (415) und das optische Element (431, 432) derartig angeordnet sind, dass das Ablationslicht (433) durch die Ultraschallvisualisierungseinheit (415) übertragbar ist.

7. Ablationsvorrichtung nach Anspruch 6, wobei die Ultraschallvisualisierungseinheit (415) eine Öffnung (434) umfasst, durch die das Ablationslicht (433) übertragbar ist.

8. Ablationsvorrichtung nach Anspruch 1, wobei
- die Ultraschallvisualisierungseinheit (15; 115; 215; 315; 415; 515; 615) dafür ausgelegt ist, ein zweidimensionales oder dreidimensionales Ultraschallbild zum Finden eines Nervs (14) einer Nierenarterie (11) hinter einer Oberfläche einer Gefäßwand der Nierenarterie (11) zu erzeugen und ein M-Mode-Ultraschallbild des Nervs (14) zum Überwachen der Ablationsprozedur zu erzeugen,
- die Ablationseinheit (19; 119; 219; 330; 431, 432; 530; 619) dafür ausgelegt ist, den Nerv (14) der Nierenarterie (11) zu abladieren,
- das Einführungselement (10) dafür ausgelegt ist, die Ultraschallvisualisierungseinheit (15; 115; 215; 315; 415; 515; 615) und die Ablationseinheit (19; 119; 219; 330; 431, 432; 530; 619) in die Nierenarterie (11) einzuführen.

9. Ablationsvorrichtung nach Anspruch 1, wobei das Einführungselement eine Hülse (541; 641) zur Einführung in das Lebewesen (7) umfasst, wobei die Hülse (541; 641) die Ablationseinheit (530; 619) umfasst und zumindest teilweise für Ultraschall transparent ist, und wobei die Ultraschallvisualisierungseinheit (515; 615) innerhalb der Hülse (541; 641) angeordnet ist, so dass Ultraschallwellen der Ultraschallvisualisierungseinheit (515; 615) durch die zumindest teilweise für Ultraschall transparente Hülse (541; 641) übertragbar sind.

10. Ablationsvorrichtung nach Anspruch 10, wobei die Ultraschallvisualisierungseinheit ein Ultraschallvisualisierungskatheter ist, der in der Hülse angeordnet werden kann.

11. Ablationscomputerprogramm zum Abladieren eines Nervs hinter einer Vorderfläche einer Gefäßwand innerhalb eines Gewebes eines Lebewesens, **dadurch gekennzeichnet, dass** das Computerprogramm Programmcodemittel umfasst, um eine Ablationsvorrichtung nach Anspruch 1 zu veranlassen, derartig betrieben zu werden, dass die zweidimensionale oder dreidimensionale Ultraschallbildgebung auf M-Mode-Ultraschallbildgebung umschaltet, wenn der Nerv in dem Gewebe gefunden wurde, wobei das M-Mode-Ultraschallbild den Nerv umfasst, die Ablationseinheit nach dem Umschalten auf M-Mode-Bildgebung dem Gewebe Ablationsenergie zuführt und die Ablationsvorrichtung die Gefäßwand kühlt, so dass durch Kühlen des Gewebes und durch Bereitstellen der Ablationsenergie eine intramurale Läsion erzeugbar ist, die sich nicht zu der Vorderfläche der Gefäßwand erstreckt, wenn das Computerprogramm auf einem Computer ausgeführt wird, der die Ablationsvorrichtung steuert.

## Revendications

1. Appareil d'ablation pour ablater un nerf à l'arrière d'une surface avant d'une paroi de vaisseau à l'intérieur d'un tissu d'un être vivant, l'appareil d'ablation comprenant :
- une unité de visualisation par ultrasons (15 ; 115 ; 215 ; 315 ; 415 ; 515 ; 615) pour générer une image ultrasonore en deux dimensions ou trois dimensions du tissu, pour trouver le nerf (14) à l'intérieur du tissu et pour générer une image ultrasonore en mode M de la structure (14) pour surveiller une procédure d'ablation pour ablater la structure à l'intérieur du tissu,
- une unité d'ablation (19 ; 119; 219; 330; 431 ; 432; 530; 619) pour fournir une énergie d'ablation au tissu,
- un élément d'introduction (10) pour introduire l'unité de visualisation par ultrasons (15 ; 115 ; 215 ; 315 ; 415 ; 515 ; 615) et l'unité d'ablation (19 ; 119 ; 219 ; 330 ; 431 ; 432 ; 530 ; 619) dans le sujet vivant (7) à proximité du tissu avec le nerf (14) devant être ablaté, et
- une unité de détermination de profondeur d'ablation (51) configurée pour déterminer une profondeur d'ablation avec le temps à partir de l'image en mode M,
**caractérisé en ce que**
- l'appareil d'ablation comprend en outre une unité de commande par ultrasons (54) configurée pour passer de l'imagerie ultrasonore bidimensionnelle ou tridimensionnelle à l'imagerie ultrasonore à mode M lorsque le nerf est trouvé à l'intérieur du tissu, l'image ultrasonore à mode M comprenant le nerf,
- l'unité d'ablation est configurée pour appliquer une énergie d'ablation au tissu après passage en imagerie en mode M,
- dans lequel l'appareil d'ablation comprend en outre une unité de refroidissement configurée pour refroidir la paroi de vaisseau de telle sorte qu'en refroidissant le tissu et en fournissant l'énergie d'ablation, une lésion intramurale peut être créée, ne s'étendant pas jusqu'à la surface avant de la paroi de vaisseau.

2. Appareil d'ablation selon la revendication 1, dans lequel l'unité de visualisation par ultrasons (215 ; 315) peut être tournée à l'intérieur de l'élément d'introduction (10) et par rapport à celui-ci.

3. Appareil d'ablation selon la revendication 1, dans lequel l'unité d'ablation et l'unité de visualisation par ultrasons sont adaptées de telle sorte que l'ablation et la visualisation par ultrasons peuvent être effectuées dans la même direction.

4. Appareil d'ablation selon la revendication 3, dans lequel l'unité d'ablation comprend une électrode d'ablation (19 ; 119; 219; 619) intégrée dans l'élément d'introduction et étant au moins partiellement transparente aux ultrasons et dans lequel l'unité de visualisation par ultrasons (15 ; 115 ; 215 ; 615) est agencée à l'intérieur de l'élément d'introduction de telle sorte que la visualisation par ultrasons peut être réalisée à travers l'électrode d'ablation (19 ; 119 ; 219 ; 619).

5. Appareil d'ablation selon la revendication 1, dans lequel l'élément d'introduction comprend une région transparente (417) transparente aux ultrasons et optiquement, dans lequel l'unité d'ablation comprend un élément optique (431, 432) pour fournir une lumière d'ablation (433) pour ablater le nerf (14), dans lequel l'élément d'introduction, l'unité de visualisation par ultrasons (415) et l'élément optique (431, 432) sont agencés de telle sorte que les ondes ultrasonores de l'unité de visualisation par ultrasons (415) et la lumière d'ablation (433) peuvent être transmises à travers la région transparente (417).

6. Appareil d'ablation selon la revendication 5, dans lequel l'unité de visualisation par ultrasons (415) est adaptée pour permettre à la lumière d'ablation (433) de traverser l'unité de visualisation par ultrasons (415), dans lequel l'unité de visualisation par ultrasons (415) et l'élément optique (431, 432) sont agencés de telle sorte que la lumière d'ablation (433) peut être transmise à travers l'unité de visualisation par ultrasons (415).

7. Appareil d'ablation selon la revendication 6, dans lequel l'unité de visualisation par ultrasons (415) comprend une ouverture (434) à travers laquelle peut être transmise la lumière d'ablation (433).

8. Appareil d'ablation selon la revendication 1, dans lequel
- l'unité de visualisation par ultrasons (15 ; 115 ; 215 ; 315 ; 415 ; 515 ; 615) est adaptée pour générer une image ultrasonore bidimensionnelle ou tridimensionnelle pour trouver un nerf (14) d'une artère rénale (11) à l'arrière d'une surface d'une paroi de vaisseau de l'artère rénale (11) et pour générer une image ultrasonore en mode M du nerf (14) pour surveiller la procédure d'ablation,
- l'unité d'ablation (19 ; 119 ; 219 ; 330 ; 431 ; 432 ; 530 ; 619) est adaptée pour ablater le nerf (14) de l'artère rénale (11),
- l'élément d'introduction (10) est adapté pour introduire l'unité de visualisation par ultrasons (15 ; 115 ; 215 ; 315 ; 415 ; 515 ; 615) et l'unité d'ablation (19 ; 119 ; 219 ; 330 ; 431 ; 432 ; 530 ; 619) dans l'artère rénale (11).

9. Appareil d'ablation selon la revendication 1, dans lequel l'élément d'introduction comporte une gaine (541; 641) devant être introduite dans le sujet vivant (7), dans lequel la gaine (541 ; 641) comprend l'unité d'ablation (530 ; 619) et est au moins partiellement transparente aux ultrasons, et dans lequel l'unité de visualisation par ultrasons (515 ; 615) est agencée à l'intérieur de la gaine (541 ; 641) de telle sorte que les ondes ultrasonores de l'unité de visualisation par ultrasons (515 ; 615) peuvent être transmises à travers la gaine au moins partiellement transparente aux ultrasons (541 ; 641).

10. Appareil d'ablation selon la revendication 10, dans lequel l'unité de visualisation par ultrasons est un cathéter de visualisation par ultrasons pouvant être agencé à l'intérieur de la gaine.

11. Programme informatique d'ablation pour ablater un nerf à l'arrière d'une surface avant d'une paroi de vaisseau à l'intérieur d'un tissu d'un être vivant, **caractérisé en ce que** le programme informatique comprend des moyens de codes de programme pour amener un appareil d'ablation tel que défini dans la revendication 1 à fonctionner de telle sorte que l'imagerie bidimensionnelle et tridimensionnelle passe à l'imagerie ultrasonore en mode M lorsque le nerf est trouvé à l'intérieur du tissu, l'image ultrasonore en mode M comprenant le nerf, l'unité d'ablation applique l'énergie d'ablation au tissu après passage en imagerie en mode M, et l'appareil d'ablation refroidit la paroi du vaisseau de telle sorte qu'en refroidissant le tissu et en fournissant l'énergie d'ablation, une lésion intramurale peut être créée, ne s'étendant pas jusqu'à la surface avant de la paroi de vaisseau, lorsque le programme informatique est exécuté sur un ordinateur commandant l'appareil d'ablation.
